# EUROPEAN PATENT SPECIFICATION

(11) **EP 2 952 949 B1**
(45) Date of publication and mention of the grant of the patent: **14.11.2018**
(21) Application number: 14745690.9
(22) Date of filing: 29.01.2014
(51) Int. Cl.: G02B 26/10, A61B 1/00, G02B 23/26, G02B 23/24

(54) **OPTICAL SCAN DEVICE**
OPTISCHE ABTASTVORRICHTUNG
DISPOSITIF DE BALAYAGE OPTIQUE

(30) Priority: 29.01.2013 JP 2013014759
(43) Date of publication of application: 09.12.2015
(73) Proprietor: Olympus Corporation, Tokyo 192-8507 (JP)
(72) Inventor: SHIMAMOTO, Atsuyoshi, Hachioji-shi, Tokyo 192-8507 (JP); NAMIKI, Mitsuru, Hachioji-shi, Tokyo 192-8507 (JP)
(74) Representative: Gunzelmann, Rainer
(86) International application number: PCT/JP2014/000458
(87) International publication number: WO 2014/119300

(56) References cited:
- EP-A1- 0 927 379
- EP-A1- 1 142 529
- JP-A- 2010 097 083
- JP-A- 2010 162 089
- JP-A- 2010 523 198
- JP-A- 2010 527 028
- US-A1- 2002 139 920

## Description

### CROSS REFERENCE TO RELATED APPLICATIONS

The present application claims the priority from Japanese Patent Application No. 2013-014759 filed on January 29, 2013.

### TECHNICAL FIELD

This disclosure relates to an optical scanning apparatus that uses an optical fiber.

### BACKGROUND

Conventionally, there has been known an optical scanning apparatus which irradiates light from an optical fiber toward an observation object to scan the observation object, and detects light reflected or scattered by the observation object or fluorescence generated in a non-observation object (see, for example, Patent Literature 1).

The optical scanning apparatus disclosed in Patent Literature 1 includes a piezoelectric actuator for vibrating the emission end of an optical fiber. The piezoelectric actuator has two pairs of piezoelectric elements for vibrating the emission end of the optical fiber in the X-direction and in the Y-direction that are orthogonal to each other, and the pair of piezoelectric elements in the X-direction and the pair of piezoelectric elements in the Y-direction are applied with voltages that are different from each other in phase by 90° while having gradually-increasing amplitude and the same frequency. As a result, the emission end of the optical fiber is spirally deflected where the vibrations in the X-direction and in the Y-direction are synthesized, to thereby spirally scan the observation object with light emitted from the optical fiber. PTL 2 discloses a medical image acquisition system including a light source, an optical fiber and a fiber deflection drive system in which the light source can emit a continuous stream of light or a stream of light pulses. PTL 3 discloses a compact display system including a viewing surface, a beam steering mechanism, a source of light remote from the viewing surface and the beam steering mechanism, and at least one waveguide connecting the source of light with the viewing surface for transmitting illumination from the source of light to the viewing surface. PTL 4 discloses an optical scanning probe system comprising a lens holder, to which a tip of the optical fiber and an object lens are fixed, a drive unit and a support member that is attached to the rear of the drive unit, the drive unit being provided with a slit, wherein a rectangular bottom plate and a side plate are formed and linked by a small linking component at the rear end.

### CITATION LIST

### Patent Literature

PTL 1: JP 2010-527028 A
PTL 2: US 2002/ 139920 A1
PTL 3: EP 0 927 379 A1
PTL 4: EP 1 142 529 A1

### SUMMARY

### (Technical Problem)

In the case of vibrating an emission end of an optical fiber, it is preferred that the vibration be at the resonance frequency of the emission end in the vibration direction, in that a larger vibration amplitude can be obtained. Further, as disclosed in Patent Literature 1, in the case of spirally scanning an observation object, the pair of the piezoelectric elements in the X-direction and the pair of the piezoelectric elements in the Y-direction are applied with voltages of the same frequency, and thus the emission end needs to have the same resonance frequency in the X-direction and in the Y-direction.

However, according to the optical scanning apparatus disclosed in Patent Literature 1, in the holding unit for holding the optical fiber held at the emission end thereof in an oscillatable manner, the optical fiber is held with a holding force that is uniform around the axis, and thus the emission end has the same resonance frequency around the axis thereof. Accordingly, when the emission end is vibrated in a first axis direction, namely in either the X-direction or the Y-direction, the emission end is easy to vibrate around the axis, which generates a trace amount of force component in a second axis direction that is orthogonal to the vibration direction, causing displacement of amplitude in the second axis direction. As a result, the scanning line tends to be elliptical rather than a straight line.

As described above, when the vibration trajectory in one axis direction becomes elliptical, the scan trajectory on the observation object becomes unstable and has distortion, making it difficult to perform observation with high precision. Such problem similarly arises when carrying out raster scanning or Lissajous scanning, without being limited to spiral scanning. The problem also arises in the case of using, not only a piezoelectric actuator, but an electromagnetic actuator including a coil and a magnet to vibrate the emission end of the optical fiber. It might be an option to carry out control to detect the vibration of the resonance frequency generated in the second axis direction so as to compensate the vibration, which however complicates the drive control of the apparatus.

In view of the aforementioned perspective, it could be helpful to provide an optical scanning apparatus capable of stably scanning an observation object with a simple configuration, without the need for complicated control.

### (Solution to Problem)

In order to solve the aforementioned problems, we provide an optical scanning apparatus having the features of claim 1. Any example or embodiment which does not fall under the scope of the claim is not part of the invention.

According to a first comparative example, the holding unit may have an adhesive for bonding the optical fiber as deflecting the optical fiber in the vibration direction caused by the driving unit and in a direction orthogonal to the vibration direction.

In the above aspect of the present invention, the holding unit has a pair of V-blocks for holding the optical fiber interposed therebetween.

According to a second comparative example holding unit may have a groove contacted by the optical fiber at four points around the axis, on an end surface having the emission end protruding therefrom.

The driving unit may be configured as a piezoelectric actuator.

### (Advantageous Effect)

The optical scanning apparatus disclosed herein is capable of stably scanning an observation object with a simple configuration, without the need for complicated control.

### BRIEF DESCRIPTION OF THE DRAWINGS

In the accompanying drawings:
FIG. 1 is a block diagram illustrating a schematic configuration of an optical scanning apparatus according to comparative example 1;
FIG. 2 is an overview schematically illustrating the optical scanning endoscope main body of FIG. 1;
FIG. 3 schematically illustrates a configuration of the tip part of the optical scanning endoscope main body of FIG. 2;
FIGS. 4 each are a view for illustrating a configuration of the scanning unit of FIG. 3;
FIG. 5 is a graph showing a holding force distribution around the axis of the illumination optical fiber of FIGS. 4;
FIG. 6 is a view illustrating a schematic configuration of a principal part of an optical scanning apparatus according to an Embodiment of the present invention; and
FIGS. 7 each are a view illustrating a schematic configuration of a principal part of an optical scanning apparatus according to comparative example 2.

The comparative examples described below only represent background that is useful for understanding the present invention, but do not form part of the same.

### DETAILED DESCRIPTION

In the following, an Embodiment of this disclosure and comparative examples are described with reference to the accompanying drawings.

### (Comparative example 1)

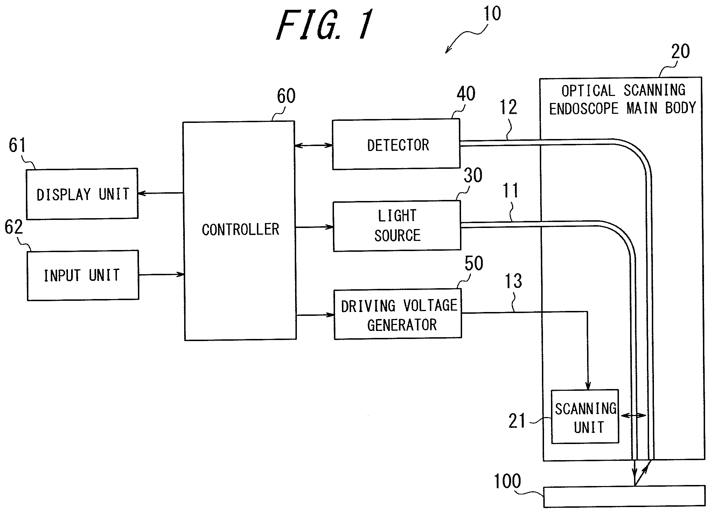

FIG. 1 is a block diagram illustrating a schematic configuration of an optical scanning apparatus according to comparative example 1. The optical scanning apparatus of FIG. 1 constitutes an optical scanning endoscope apparatus 10, and includes: an optical scanning endoscope main body 20, a light source 30, a detector 40, a driving voltage generator 50, a controller 60, a display unit 61, and an input unit 62. The light source 30 and the optical scanning endoscope main body 20 are optically coupled to each other via an illumination optical fiber 11 formed of, for example, a single mode fiber. The detector 40 and the optical scanning endoscope main body 20 are optically coupled to each other via a detection optical fiber bundle 12 formed of, for example, multimode fibers. Here, the light source 30, the detector 40, the driving voltage generator 50, and the controller 60 may all be accommodated in the same housing or may independently be accommodated in separate housings.

The light source 30 multiplexes light from three laser sources emitting CW (continuous wave) laser light in three primary colors of red, green, and blue, and emits the multiplexed light as white light. DPSS lasers (diode-pumped solid-state laser) and laser diodes, for example, may suitably be used as the laser source. It is obvious, however, that the configuration of the light source 30 is not limited thereto, and may use one laser light source or a plurality of other light sources.

The optical scanning endoscope main body 20 irradiates an observation object 100 with light emitted from the light source 30 via an illumination optical fiber 11, while vibrating the emission end of the illumination optical fiber 11 by means of a scanning unit 21, so as to two-dimensionally scan (spirally scan in comparative example 1) the observation object 100, condenses signal light obtained through the scanning, and transmits the condensed light to the detector 40 via the detection optical fiber bundle 12. Here, the driving voltage generator 50 feeds a necessary vibrating voltage to the scanning unit 21 via a wiring cable 13, based on control from the controller 60.

The detector 40 separates signal light transmitted through the detection optical fiber bundle 12 into spectral components, and photoelectrically converts, into an electric signal, the signal light thus separated. The controller 60 synchronously controls the light source 30, the detector 40, and the driving voltage generator 50, while processing electric signals output by the detector 40 so as to display an image on the display unit 61. The controller 60 also makes various settings as to the scanning speed, the brightness of an image to be displayed, and the like, based on an input signal generated though an input operation from the input unit 62.

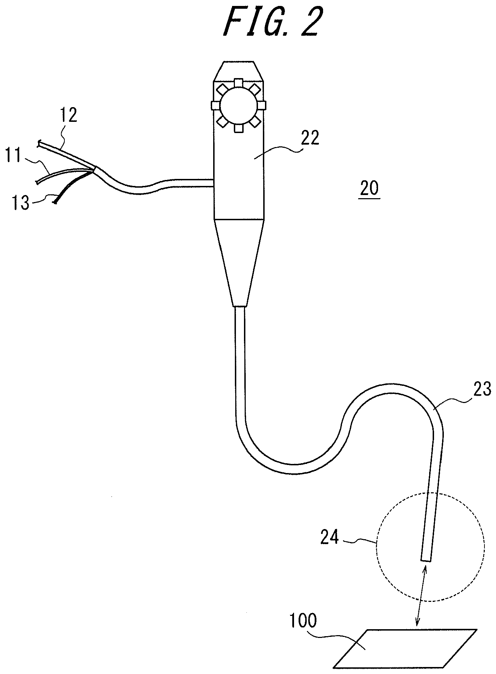

FIG. 2 is an overview schematically illustrating the optical scanning endoscope main body 20. The optical scanning endoscope main body 20 includes an operation portion 22 and a flexible insertion portion 23. The illumination optical fiber 11 coupled to the light source 30, the detection optical fiber bundle 12 coupled to the detector 40, and the wiring cable 13 connected to the driving voltage generator 50 are each guided through inside the insertion portion 23 to the tip part 24 (part enclosed by the dashed line of FIG. 2). The tip part 24 is subjected to bending operation by means of the operation portion 22.

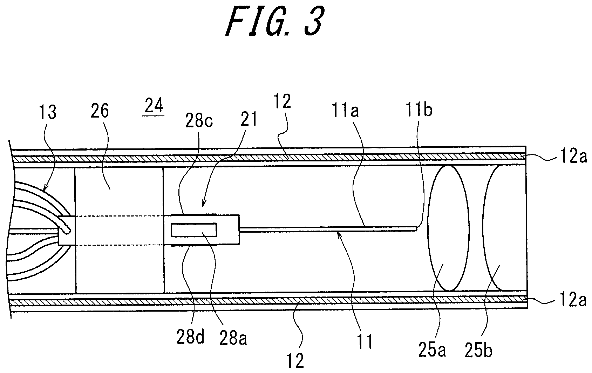

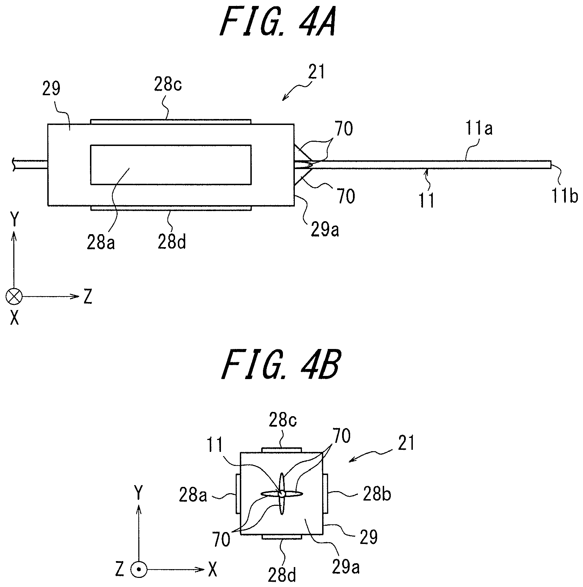

FIG. 3 schematically illustrates a configuration of the tip part 24 of the optical scanning endoscope main body 20 of FIG. 2. FIG. 4(a) is an enlarged view of the scanning unit 21 of FIG. 3, and FIG. 4(b) is a right-side view of FIG. 4(a). The tip part 24 is provided with the scanning unit 21, projection lenses 25a, 25b, and a detection lens (not shown), and has the illumination optical fiber 11 and the detection optical fiber bundle 12 extending therethrough.

The scanning unit 21 includes a prism-shaped fiber holder 29 fixed within the insertion portion 23 by means of a mounting ring 26. The illumination optical fiber 11 passes through the center of the fiber holder 29 and supported by the fiber holder 29 in such a manner as to allow an emission end 11a to oscillate. The four side surfaces of the fiber holder 29 face the X-direction (first direction) or the Y-direction (second direction) each being orthogonal to the axial direction (Z-direction) of the illumination optical fiber 11 held by the fiber holder 29.

Fixed on the side surfaces facing the X-direction of the fiber holder 29 is a pair of X-direction-drive piezoelectric elements 28a, 28b for vibrating the emission end 11a in the X-direction. Meanwhile, fixed on the side surfaces facing the Y-direction of the fiber holder 29 is a pair of Y-direction-drive piezoelectric elements 28c, 28d for vibrating the emission end 11a in the Y-direction. The piezoelectric elements 28a to 28d are connected to the driving voltage generator 50 via the wiring cable 13. That is, the emission end 11a is driven to vibrate in the X-direction and in the Y-direction by a driving unit formed of a piezoelectric actuator having piezoelectric elements 28a to 28d. Meanwhile, the detection optical fiber bundle 12 is disposed so as to pass though the outer periphery of the tip part 24.

The projection lenses 25a, 25b and the detection lens are disposed in the vicinity of the tip surface of the tip part 24. The projection lenses 25a, 25b are configured such that laser light emitted from the emission end surface 11b of the illumination optical fiber 11 is substantially condensed onto the observation object 100. The detection lens is arranged in such a manner as to take in, as detection light, the laser light that has been condensed onto the observation object 100 and then reflected, scattered, and refracted by the observation object 100 (light that has been interacted with the observation object 100) or fluorescence, so as to have the detection light condensed onto and coupled to the detection optical fiber bundle 12 disposed in the subsequent stage of the detection lens. The number of the projection lenses is not limited two, and the projection lens(es) may be composed of one lens or a plurality of lenses.

In comparative example 1, the illumination optical fiber 11 is further bonded, as illustrated in FIGS. 4(a) and 4(b), through an adhesive 70 at an end surface 29a of the fiber holder 29 where the emission end 11a is positioned. The adhesive 70 is provided such that the emission end 11a is bonded to the fiber holder 29, where the adhesive 70 is unevenly distributed in the vibration directions of the emission end 11a, namely, in the X-direction and in the Y-direction. In other words, the holding structure of the illumination optical fiber 11 with respect to the fiber holder 29 is different, around the axis of the illumination optical fiber 11, between the vibration direction/a direction orthogonal to the vibration direction, and other directions, which means that the illumination optical fiber 11 is held by the fiber holder 29 with a holding force that is axially nonuniform.

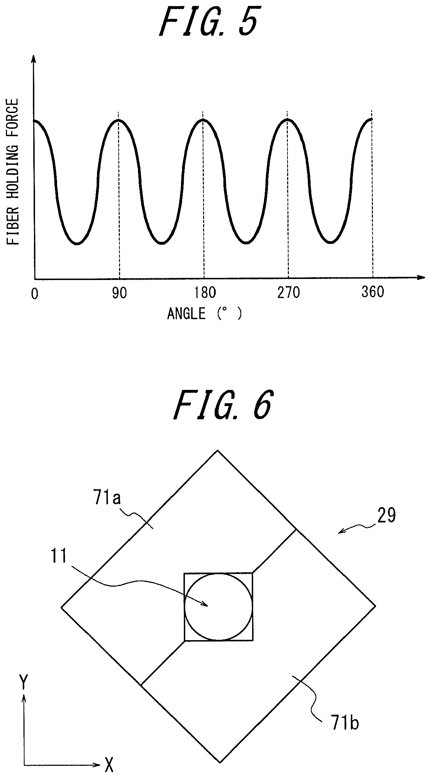

In comparative example 1, the fiber holder 29 holds the emission end 11a of
the illumination optical fiber 11 with a holding force that is distributed nonuniformly, as illustrated in FIG. 5, around the optical axis O of the illumination optical fiber 11, where the +X-direction from the optical axis O of FIG. 4(b) is defined as 0°. More specifically, the holding force is nonuniformly distributed so as to have peak values that become substantially equal to each other in the vibration directions, i.e., in the X-direction (0°, 180°) and the Y-direction (90°, 270°). Further, the resonance frequencies of the emission end 11a in the X-direction and in the Y-direction are the same or substantially the same, and thus substantially equal to each other.

With the aforementioned configuration, when carrying out observation by using the optical scanning endoscope apparatus 10, the controller 60 controls the driving voltage generator 50 to apply, via the wiring cable 13, a vibration voltage to the piezoelectric elements 28a to 28d of the scanning unit 21. Here, the X-direction-drive piezoelectric elements 28a, 28b are applied with a vibration voltage having a drive frequency substantially equal to the resonance frequency of the emission end 11a in the X-direction and an amplitude that gradually increases. The Y-direction-drive piezoelectric elements 28c, 28d are applied with a vibration voltage that is the same as the vibration voltage in the X-direction but different in phase by 90°. As a result, the emission end surface 11b of the illumination optical fiber 11 is spirally deflected.

The controller 60 applies, by means of the driving voltage generator 50, a voltage to the piezoelectric elements 28a to 28d while driving the light source 30. As a result, laser light emitted from the light source 30 travels through the illumination optical fiber 11 so as to be irradiated onto the observation object 100 via the emission end surface 11b so as to spirally scan the observation object 100.

The irradiation of laser light onto the observation object 100 provides reflected light, scattered light, and light generated from the observation object 100, which are condensed by the detection lens as detection light and caused to incident on the incidence end surface 12a of the detection optical fiber bundle 12. The detection light is guided through the detection optical fiber bundle 12 to the detector 40, and detected in the detector 40 for each wavelength component.

The controller 60 calculates, based on the amplitude of a driving voltage applied to the piezoelectric elements 28a to 28d from the driving
voltage generator 50, information on the scanning position on the scanning path, while obtaining, based on an electric signal output from the detector 40, pixel data on the observation object 100 at the scanning position. The controller 60 sequentially stores, in a memory (not shown), information on the scanning position and the pixel data, subjects the information to necessary processing such as interpolation processing after completing the scanning or during the scanning, so as to generate an image of the observation object 100, and displays the image on the display unit 61.

Here, the fiber holder 29 holds the emission end 11a of the illumination optical fiber 11 with a holding force that is nonuniform around the axis of the illumination optical fiber 11 due to the adhesive 70 as illustrated in FIG. 5, with having peaks in the vibration directions, namely, in the X-direction and in the Y-direction. Therefore, according to comparative example 1, when the emission end 11a is vibrated in the X-direction, the emission end 11a becomes less likely to vibrate around the axis. Similarly, when the emission end 11a is vibrated in the Y-direction, the emission end 11a becomes less likely to vibrate around the Z-axis. As a result, the emission end 11a is spirally deflected with accuracy, which allows for stable spiral scan of the observation object 100 with a simple configuration, without the need for complicated control.

### (Embodiment)

FIG. 6 is a view illustrating a schematic configuration of a principal part of an optical scanning apparatus according to an Embodiment of the present invention. According to the Embodiment, in the scanning unit 21 of the optical scanning endoscope apparatus 10 of comparative example 1, the fiber holder 29 for holding, in an oscillatable manner, the emission end 11a of the illumination optical fiber 11 includes a pair of V-blocks 71a, 71b for holding the illumination optical fiber 11 interposed therebetween.

With the aforementioned configuration, the illumination optical fiber 11 is held around the axis as being in contact with the V-blocks 71a, 71b at four points, and thus the holding force to be exerted by the V-blocks 71a, 71b becomes nonuniform around the axis. More specifically, a mechanism for holding the illumination optical fiber 11 in the vibration direction/a direction orthogonal to the vibration direction is different from the mechanism in other directions. Therefore, the X-direction and the Y-direction in which the illumination optical fiber 11 contacts with the V-blocks 71a, 71b may each be
defined as the vibratory drive direction, so that the resonance frequencies can be made substantially equal to each other, to thereby obtain an effect similar to that of comparative example 1. The piezoelectric elements constituting the piezoelectric actuator may be mounted onto the V-blocks 71a, 71b through mounting surfaces which may be flattened as appropriate.

### (Comparative example 2)

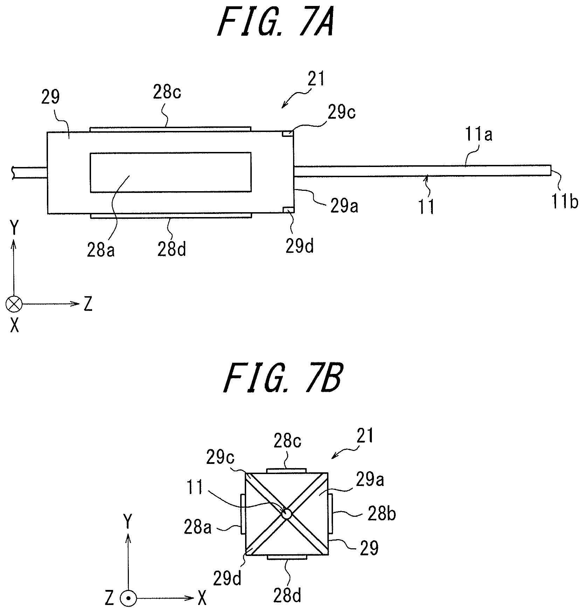

FIGS. 7 each are a view illustrating a schematic configuration of the principal part of an optical scanning apparatus according to comparative example 2. According to comparative example 2, in the scanning unit 21 of the optical scanning endoscope apparatus 10 of comparative example 1, the fiber holder 29 for holding, in an oscillatable manner, the emission end 11a of the illumination optical fiber 11 has grooves 29c, 29d formed in the end surface 29a in such a manner that the illumination optical fiber 11 contacts therewith at four points around the axis. Here, FIG. 7(a) is an enlarged view of the fiber holder 29, and FIG. 7(b) is a right-side view of FIG. 7(a).

With the aforementioned configuration, the illumination optical fiber 11 contacts with the end surface 29a of the fiber holder 29 at four points around the axis where the groove 29c and 29d intersect with each other, which makes the holding force of the fiber holder 29 nonuniform around the axis. More specifically, a mechanism for holding the illumination optical fiber 11 in the vibration direction and a direction orthogonal to the vibration direction is different from the mechanism in other directions. Therefore, the X-direction and the Y-direction in which the illumination optical fiber 11 contacts with the end surface 29a may each be defined as the vibratory drive direction, so that the resonance frequencies can be made substantially equal to each other, to thereby obtain an effect similar to that of comparative example 1.

It should be noted that this disclosure is not limited to the aforementioned Embodiment, and various modifications and alterations can be made thereto. For example, in the aforementioned Embodiment, the fiber holder 29 is configured to hold the illumination optical fiber 11 with a holding force that increases in the X-direction and in the Y-direction being the vibration directions orthogonal to each other. However, in the aforementioned Embodiment, the vibration directions each may be in a direction rotated in the X-Y plane by 45 degrees from the X-direction or from the Y-direction, in which the holding force reduces. Further, the shape of the fiber holder 29 is not limited to a prism shape, and may be a columnar shape.

The scanning of the observation object 100 by means of the illumination optical fiber 11 is not limited to spiral scanning, and may be raster scanning, Lissajous scanning, or the like. The generation of undesired vibration components in a direction orthogonal to the vibration direction can still be prevented in this case as well, making it possible to stably scan the observation object. In the case of raster scanning, the resonance frequency in the X-direction may be set higher than the resonance frequency in the Y-direction, so as to effect the vibration at a drive frequency corresponding to each of the resonance frequencies.

Further, the driving unit for vibrating the emission end 11a is not limited to a piezoelectric actuator, and may employ an electromagnetic actuator having a coil and a magnet. This disclosure is effectively applicable, not only to an optical scanning endoscope apparatus but other optical scanning apparatuses such as a microscope.

### REFERENCE SIGNS LIST

- 10: optical scanning endoscope apparatus
- 11: illumination optical fiber
- 11a: emission end
- 11b: emission end surface
- 12: detection optical fiber bundle
- 13: wiring cable
- 20: optical scanning endoscope main body
- 21: scanning unit
- 22: operation portion
- 23: insertion portion
- 24: tip part
- 25a, 25b: projection lens
- 26: mounting ring
- 28a to 28d: piezoelectric element
- 29: fiber holder
- 29a: end surface
- 29c, 29d: groove
- 30: light source
- 40: detector
- 50: driving voltage generator
- 60: controller
- 61: display unit
- 62: input unit
- 70: adhesive
- 71a, 71b: V-block
- 100: observation object

## Claims

1. An optical scanning apparatus (10), comprising:
an optical fiber (11);
a holding unit (29) adapted to hold an emission end (11a) of the optical fiber (11) in an oscillatable manner, the optical fiber (11) being configured to pass through the center of the holding unit (29); and
a driving unit (28a, 28b, 28c, 28d) configured to drive the emission end (11a) and including piezoelectric actuators, wherein
a first pair of piezoelectric actuators (28a, 28c) is fixed on side surfaces facing a first direction of the holding unit (29) such that the emission end (11a) of the optical fiber (11) can be vibrated in the first direction and wherein a second pair of piezoelectric actuators (28b, 28d) is fixed on side surfaces facing a second direction of the holding unit (29) such that the emission end (11a) of the optical fiber (11) can be vibrated in the second direction, wherein the first direction, the second direction and the axial direction of the optical fiber are each orthogonal to each other,
**characterized in that**
the holding unit (29) has a pair of V-blocks (71a, 71b) for holding the optical fiber (11) interposed therebetween;
the optical fiber (11) is held around its axial direction as being in contact with the V-blocks (71a, 71b) at four points.

## Patentansprüche

1. Optische Abtastvorrichtung (10), umfassend:
einen Lichtleiter (11);
eine Halteeinheit (29), die eingerichtet ist ein Ausstrahlende (11a) des Lichtleiters (11) in einer oszillierbaren Art zu halten, wobei der Lichtleiter (11) dazu konfiguriert ist, durch das Zentrum der Halteeinheit (29) zu verlaufen; und
eine Antriebseinheit (28a, 28b, 28c, 28d), die konfiguriert ist, um das Ausstrahlende (11 A) anzutreiben, und die piezoelektrische Stellantriebe beinhaltet, wobei
ein erstes Paar von piezoelektrischen Stellantrieben (28a, 28c) auf Seitenoberflächen, die einer ersten Richtung der Halteeinheit (29) zugewandt sind, befestigt ist, so dass das Ausstrahlende (11a) des Lichtleiters (11) in der ersten Richtung geschwungen werden kann und wobei ein zweites Paar von elektronischen Stellantrieben (28b, 28d) auf Seitenoberflächen, die einer zweiten Richtung der Halteeinheit (29) zugewandt sind, befestigt ist, so dass das Ausstrahlende (11a) des Lichtleiters (11) in die zweite Richtung geschwungen werden kann, wobei die erste Richtung, die zweite Richtung und die axiale Richtung des Lichtleiters jeweils orthogonal zueinander sind,
**dadurch gekennzeichnet, dass**
die Halteeinheit (29) ein Paar V-Blöcke (71a, 71b) zum Halten des dazwischenliegenden Lichtleiters (11) aufweist;
der Lichtleiter (11) um seine axiale Richtung gehalten wird, so dass er die V-Blöcke (71a, 71b) an vier Punkten kontaktiert.

## Revendications

1. Appareil de balayage optique (10), comprenant :
une fibre optique (11) ;
une unité de maintien (29) conçue pour maintenir une extrémité d'émission (11a) de la fibre optique (11) de manière à pouvoir osciller, la fibre optique (11) étant conçue pour traverser le centre de l'unité de maintien (29) ; et
une unité de commande (28a, 28b, 28c, 28d) conçue pour entraîner l'extrémité d'émission (11a) et comprenant des actionneurs piézoélectriques,
dans lequel
une première paire d'actionneurs piézoélectriques (28a, 28c) est fixée sur des surfaces latérales faisant face à une première direction de l'unité de maintien (29) de sorte que l'extrémité d'émission (11a) de la fibre optique (11) puisse vibrer dans la première direction et dans lequel une seconde paire d'actionneurs piézoélectriques (28b, 28d) est fixée sur des surfaces latérales faisant face à une seconde direction de l'unité de maintien (29) de sorte que l'extrémité d'émission (11a) de la fibre optique (11) puisse vibrer dans la seconde direction, dans lequel la première direction, la seconde direction et la direction axiale de la fibre optique sont chacune orthogonales l'une à l'autre,
**caractérisé en ce que**
l'unité de maintien (29) comporte une paire de blocs en V (71a, 71b) destinés à maintenir la fibre optique (11) interposée entre eux ;
la fibre optique (11) est maintenue autour de sa direction axiale étant donné qu'elle est en contact avec les blocs en V (71a, 71b) au niveau de quatre points.
